# EUROPEAN PATENT APPLICATION

(11) **EP 4 644 397 A1**
(43) Date of publication of application: **05.11.2025**
(21) Application number: 23911984.5
(22) Date of filing: 22.12.2023
(51) Int. Cl.: C07F 7/00, C07C 61/22, C07F 9/94, G03F 7/004, G03F 7/20, G03F 7/32

(54) **METALLIC COMPOUND, PHOTOSENSITIVE COMPOSITION, PATTERN FORMATION METHOD, AND SUBSTRATE MANUFACTURING METHOD**

(30) Priority: 26.12.2022 JP 2022208687; 29.09.2023 JP 2023169294
(71) Applicant: Mitsubishi Chemical Corporation, Tokyo 100-8251 (JP); National Institutes for Quantum Science and Technology, Chiba 263-8555 (JP)
(72) Inventor: HATTORI, Shigeki, Tokyo 100-8251 (JP); NAGAYAMA, Kazuhiro, Tokyo 100-8251 (JP); KATO, Akane, Tokyo 100-8251 (JP); YAMAMOTO, Hiroki, Chiba-shi, Chiba 263-8555 (JP)
(74) Representative: Vossius & Partner Patentanwälte Rechtsanwälte mbB
(86) International application number: PCT/JP2023/046175
(87) International publication number: WO 2024/143204

(57) **Abstract**

The present invention provides a compound capable of realizing fine circuit patterns, a photosensitive composition containing the same, and a pattern forming method using the photosensitive composition. The pattern forming method uses a photosensitive composition comprising a compound composed of a transition metal or a poor metal element and of a carboxy ligand including an alicyclic structure having a double bond.

## Description

### TECHNICAL FIELD

The present invention relates to a metal compound suitable for use in ultra-microlithography processes, such as the production of ultra-LSIs and high-capacity microchips, and other photofabrication processes, a photosensitive composition containing the same, and a pattern forming method using the photosensitive composition.

### BACKGROUND ART

Conventionally, in the production process of semiconductor devices, lithography fine processing is performed by using photoresist compositions.

In semiconductor photolithography, in accordance with Moore's Law, circuit patterns become smaller as semiconductor devices become more miniaturized, and further miniaturization is desired.

Photolithography is broadly based on the shortening of wavelengths of light sources in exposure devices and the use of photoresists that respond to this. Photoresists are required to meet all of the requirements of high resolution, low roughness, and high sensitivity. Conventional resists that contain photoacid generators, known as chemically amplified resists, cause a decrease in resolution due to diffusion of acid, and are therefore considered incapable of handling ultra-fine patterns.

In recent years, non-chemically amplified photoresists based on compounds containing metal elements such as Zn and Sn have been proposed, and it has been reported that they can be used to form fine patterns using next-generation exposure devices that use extreme ultraviolet (EUV) rays.

For example, Patent Literatures 1 to 5 and Non-Patent Literatures 1 to 3 listed below disclose methods of forming resist patterns using extreme ultraviolet (EUV) rays or electron beams.

In addition, Patent Literatures 6 and 7 disclose photoresists containing poor metal elements such as bismuth.

### CITATION LIST

### PATENT LITERATURE

Patent Literature 1: Japanese Patent Laid-Open No. 2015-108781
Patent Literature 2: Japanese Patent Laid-Open No. 2001-072716
Patent Literature 3: Japanese Patent Laid-Open No. 2017-173537
Patent Literature 4: Japanese Patent Laid-Open No. 2012-185484
Patent Literature 5: Japanese Patent Laid-Open No. 2021-102604
Patent Literature 6: Japanese Patent Laid-Open No. 2011-253185
Patent Literature 7: Japanese Patent Laid-Open No. 2023-013561

### NON-PATENT LITERATURE

Non-Patent Literature 1: Minoru Toriumi etc., Proc. SPIE, 9779 (2016) 97790G
Non-Patent Literature 2: Lianjia Wu etc., Proc. SPIE, 10957 (2019) 109570B
Non-Patent Literature 3: Neha Thakur etc., Proc. SPIE, 10957 (2019) 10957D

### SUMMARY OF THE INVENTION

### PROBLEM TO BE SOLVED BY THE INVENTION

In the field of photolithography, especially semiconductor photolithography, there is a need for photosensitive compositions and pattern forming methods that can achieve further miniaturization of circuit patterns.

The present inventors have found, as a result of intensive study, that a compound containing a transition metal or a poor metal and a carboxy ligand including an alicyclic structure having a double bond is suitable as a photoresist for handling ultra-fine patterns. In addition, the present inventors have found that a metal cluster compound composed of a transition metal element and a carboxy ligand including an alicyclic structure having a double bond is suitable.

Therefore, an object of the present invention is to provide a transition metal compound or a poor metal compound capable of achieving miniaturization of circuit patterns, a photosensitive composition containing the same, and a pattern forming method using the photosensitive composition.

### MEANS FOR SOLVING PROBLEM

The present invention has the following aspects.
[1] A transition metal cluster compound comprising a transition metal element and a carboxy ligand including an alicyclic structure having a double bond, wherein the compound forms a cluster centered on the transition metal element.
[2] The transition metal cluster compound according to [1], wherein the carboxy ligand including an alicyclic structure having a double bond comprises a ligand represented by the following general formula (1). 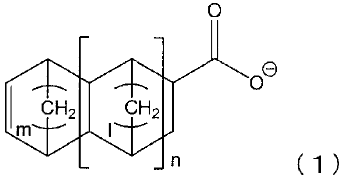 wherein n represents an integer of 0 or 1 or more, and m and l each represent an integer of 1 or more and may be the same or different.
[3] The transition metal cluster compound according to [1], wherein the carboxy ligand including an alicyclic structure having a double bond comprises a ligand represented by the following general formula (2). 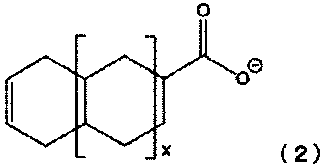 wherein x represents an integer of 0 or 1 or more.
[4] The transition metal cluster compound according to any one of [1] to [3], wherein the transition metal element comprises one or more elements selected from the group consisting of zirconium, hafnium, and titanium.
[5] The transition metal cluster compound according to any one of [1] to [4], comprising 2 or more and 6 or less of the transition metal elements.
[6] A method for producing the transition metal cluster compound according to [2] or [3], wherein a carboxylic acid having a structure represented by the general formula (1) or (2) is reacted with a compound containing a transition metal element in a solution.
[7] A method for producing the transition metal cluster compound according to [2] or [3], wherein a carboxylic acid having a structure represented by the general formula (1) or (2) is reacted with a compound containing a transition metal element in a solution in the presence of water.
[8] A method for producing the transition metal cluster compound according to [2] or [3], wherein a carboxylic acid having a structure represented by the general formula (1) or (2) is mixed into an alcohol solution of a compound containing a transition metal element and reacted in the solution.
[9] A method for producing the transition metal cluster compound according to [2] or [3], wherein a carboxylic acid having a structure represented by the general formula (1) or (2) is reacted with an alcohol solution of an alkoxide of the transition metal element.
[10] A photosensitive composition comprising the transition metal cluster compound according to any one of [1] to [5].
[11] The photosensitive composition according to [10], wherein the transition metal cluster compound accounts for 50% by mass or more of components of the photosensitive composition excluding a solvent.
[12] The photosensitive composition according to [10] or [11], wherein the photosensitive composition reacts with light having a wavelength of 6.5 to 13.5 nm.
[13] A pattern forming method comprising a step of coating the photosensitive composition according to any one of [10] to [12] onto a substrate, a step of exposing the composition to chemical radiation, and a step of developing the composition using a developer.
[14] The pattern forming method according to [13], wherein the developer comprises an organic solvent having a solubility parameter (SP value) of 7.5 to 11.
[15] A method for producing a substrate having a patterned layer obtained by the pattern forming method according to [13] or [14].
[16] A complex compound comprising a poor metal element and a carboxy ligand including an alicyclic structure having a double bond.
[17] The complex compound according to [16], wherein the poor metal element comprises arsenic, antimony, or bismuth.
[18] The complex compound according to [16], wherein the carboxy ligand including an alicyclic structure having a double bond comprises a ligand represented by the following general formula (1) or (2). 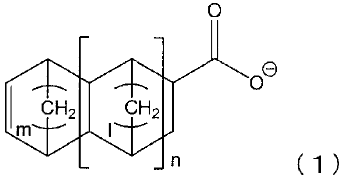
   wherein n represents an integer of 0 or 1 or more, and m and l each represent an integer of 1 or more and may be the same or different. 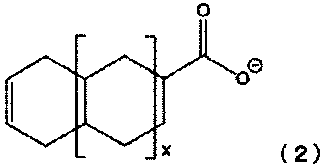
   wherein x represents an integer of 0 or 1 or more.
[19] The complex compound according to any one of [16] to [18], comprising a compound represented by the following general formula (3) or (4). 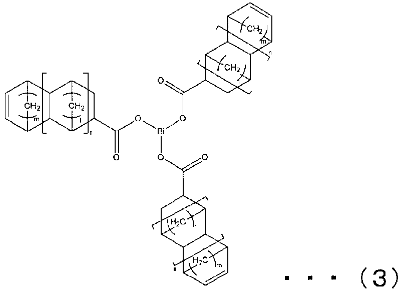
   wherein n represents an integer of 0 or 1 or more, and m and l each represent an integer of 1 or more and may be the same or different. 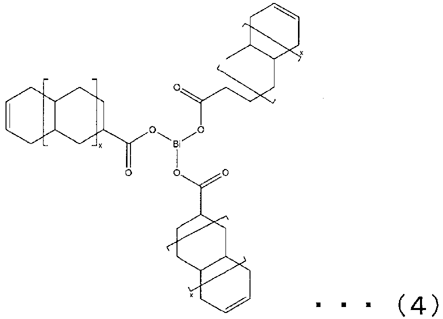
   wherein x represents an integer of 0 or 1 or more.
[20] A method for producing the complex compound according to [18], wherein a carboxylic acid having a structure represented by the general formula (1) or (2) is reacted with a compound containing a poor metal element using a hydrocarbon-based solvent.
[21] A method for producing the complex compound according to [18], wherein a carboxylic acid having a structure represented by the general formula (1) or (2) is reacted with a bismuth compound using a hydrocarbon-based solvent.
[22] A photosensitive composition comprising the complex compound according to any one of [16] to [19].
[23] The photosensitive composition according to [22], wherein the complex compound accounts for 50% by mass or more of components of the photosensitive composition excluding a solvent.
[24] The photosensitive composition according to [22] or [23], wherein the photosensitive composition reacts with light having a wavelength of 6.5 to 13.5 nm.
[25] A pattern forming method comprising a step of coating the photosensitive composition according to any one of [22] to [24] onto a substrate, a step of exposing the composition to chemical radiation, and a step of developing the composition using a developer.
[26] The pattern forming method according to [25], wherein the developer comprises an organic solvent having a solubility parameter (SP value) of 7.5 to 11.
[27] A method for producing a substrate having a patterned layer obtained by the pattern forming method according to [25] or [26].
[28] A photosensitive composition comprising a compound composed of a transition metal or a poor metal element and of a carboxy ligand including an alicyclic structure having a double bond.
[29] A pattern forming method comprising a step of coating the photosensitive composition according to [28] onto a substrate, a step of exposing the composition to chemical radiation, and a step of developing the composition using a developer.

### EFFECT OF THE INVENTION

The transition metal cluster compound, complex compound, and photosensitive composition containing the same according to the present invention form a negative-type pattern by being polymerized and rendered insoluble in an organic solvent by the reaction of a double bond in a ligand upon absorption of light energy after exposure to extreme ultraviolet (EUV) rays. It is believed that the insoluble material that is formed by the reaction and polymerization of the double bond becomes amorphous, which suppresses roughness and allows the formation of a high-resolution pattern, and that the insoluble material is more likely to become amorphous when it has a ligand having an alicyclic structure.

Therefore, by having a carboxy ligand including an alicyclic structure having a double bond, a pattern with low-roughness and high-resolution can be obtained.

It is also highly sensitive to extreme ultraviolet (EUV) exposure, and can, for example, form a half-pitch (hp) 100 nm or 50 nm L&S (1 : 1) pattern with high resolution in an electron beam (EB) lithography test.

In addition, the use of transition metal elements such as zirconium, hafnium, and titanium in the compound according to the present invention is desirable because they are likely to generate secondary electrons upon exposure to extreme ultraviolet (EUV) rays, and the generated secondary electrons induce a reaction of the double bond in the ligand. It is also desirable to use poor metal elements such as bismuth in the compound according to the present invention because they easily absorb extreme ultraviolet (EUV) rays.

### MODE(S) FOR CARRYING OUT THE INVENTION

The following is an explanation of the present invention based on embodiments. However, the present invention is not limited to the embodiments.

As used herein, the numerical range in the description where "to" is used as a notation expression to represent the numerical range from the lower limit to the upper limit refers to a numerical range specified as being equal to or more than the lower limit value and equal to or less than the upper limit value, including the lower limit value and the upper limit value.

### [Compound Composed of Transition Metal Element or Poor Metal Element and Carboxy Ligand Including Alicyclic Structure Having Double Bond]

A compound according to one embodiment of the present invention is a compound composed of a transition metal element or a poor metal element and a carboxy ligand including an alicyclic structure having a double bond, and is, for example, a transition metal cluster compound (hereinafter also referred to as "the present cluster compound") composed of a transition metal element and a carboxy ligand including an alicyclic structure having a double bond, which forms a cluster centered on the transition metal element. In the present invention, the cluster compound means a metal complex molecule having a metal atom and a ligand, in which a plurality of metal atoms are bonded to each other directly or through a bridging ligand. The present cluster compound may contain oxygen and/or hydroxyl groups in the structure, and may preferably contain µ-oxo ligands (µ-O) and/or µ-hydroxy groups (µ-OH).

The transition metal element in the present cluster compound is preferably one or more elements selected from zirconium, hafnium, and titanium, of which zirconium or hafnium is preferred. Hafnium is an element in the same group as zirconium and has very similar chemical and physical properties.

The present cluster compound preferably contains 2 or more and 6 or less transition metal elements, more preferably 4 or more and 6 or less, and particularly preferably 5 or more and 6 or less, in one molecule.

Another example of the compound according to one embodiment of the present invention is a poor metal complex compound (hereinafter also referred to as "the present poor metal complex compound") composed of a poor metal element and a carboxy ligand including an alicyclic structure having a double bond. Preferably, it is composed of a poor metal element such as arsenic, antimony, or bismuth, and a carboxy ligand including an alicyclic structure having a double bond.

The poor metal element in the present poor metal compound is preferably arsenic, antimony, or bismuth, particularly preferably bismuth.

The number of types of poor metal elements in one molecule of the complex compound composed of such a poor metal element and a carboxy ligand including an alicyclic structure having a double bond is preferably 1 or more and 10 or less, more preferably 1 or more and 6 or less, and particularly preferably 1, i.e., a mononuclear complex compound.

The carboxy ligand including an alicyclic structure having a double bond has a cycloalkenyl group, and is preferably, for example, a ligand represented by the following general formula (1) or (2). 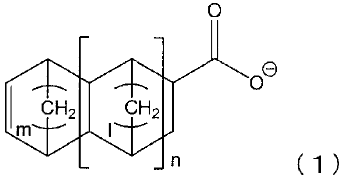
wherein n represents an integer of 0 or 1 or more, and m and l each represent an integer of 1 or more and may be the same or different. 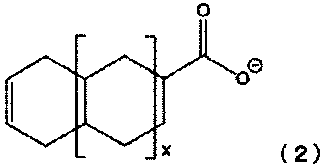
wherein x represents an integer of 0 or 1 or more.

In the general formula (1), n is preferably 0 to 2, more preferably 0 to 1; m is preferably 1 to 3, more preferably 1 to 2; and l is preferably 1 to 3, more preferably 1 to 2. In addition, m and l may be the same or different.

In the general formula (2), x is preferably 0 to 2, more preferably 0 to 1.

The structure of the ligand can be analyzed by NMR.

The molecular weight of the present cluster compound is preferably 1,000 to 5,000, more preferably 1,000 to 4,000, and even more preferably 1,500 to 3,000.

When the molecular weight of the present cluster compound is equal to or less than the upper limit, the volume becomes small, and thus an improvement in roughness and resolution is expected, while when the molecular weight is equal to or more than the lower limit, the coating properties and etching resistance tend to be improved. However, the molecular weight described above is only a guide and is not intended to be limiting, as it does not determine the lithographic properties alone.

The molecular weight of the present poor metal complex compound is preferably 1,000 to 5,000, more preferably 1,000 to 4,000, and even more preferably 1,500 to 3,000.

When the molecular weight is equal to or less than the upper limit, the volume becomes small, and thus an improvement in roughness and resolution is expected, while when the molecular weight is equal to or more than the lower limit, the coating properties and etching resistance tend to be improved. However, the molecular weight described above is only a guide and is not intended to be limiting, as it does not determine the lithographic properties alone.

Examples of the present poor metal complex compound include compounds represented by the following general formula (3) or (4). 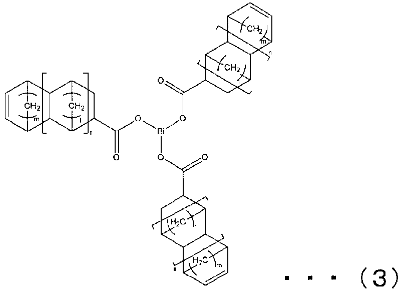 wherein n represents an integer of 0 or 1 or more, and m and l each represent an integer of 1 or more and may be the same or different. 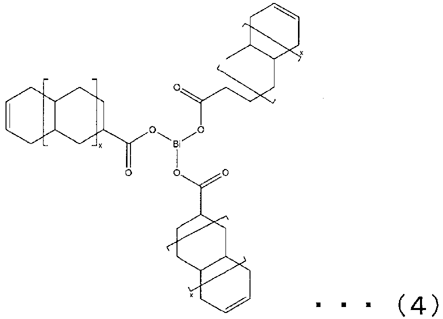 wherein x represents an integer of 0 or 1 or more.

### [Production Method]

The present cluster compound can be produced, for example, by reacting a carboxylic acid having a structure represented by the general formula (1) or (2) with a compound containing a transition metal element in a solution. Preferably, it is a method in which a carboxylic acid having a structure represented by the general formula (1) or (2) is reacted with a compound containing a transition metal element in a solution in the presence of water. Preferably, it is a method in which a carboxylic acid having a structure represented by the general formula (1) or (2) is mixed into an alcohol solution of a compound containing a transition metal element and reacted in the solution. Preferably, the method for producing a transition metal cluster compound is a method in which a carboxylic acid having a structure represented by the general formula (1) or (2) is reacted with an alcohol solution of an alkoxide of the transition metal element.

More specifically, for example, a carboxylic acid having a structure represented by the general formula (1) or (2) and an alcohol solution of an alkoxide of the transition metal element are mixed in a reaction vessel, and the mixture is stirred and then stored for preferably 1 to 24 hours, more preferably 3 to 24 hours. During the above process, it is preferable to perform the reaction in the presence of water. Such water may be a by-product of the reaction of alcohol present in the system and the carboxylic acid, or water that is intentionally added.

The precipitated crystals can then be filtered to obtain the present cluster compound.

The storage temperature is preferably room temperature, and specifically, it is preferably 15°C to 40°C, more preferably 20°C to 30°C.

The reaction vessel is preferably a sealed vessel, and for small amounts, for example, a Schlenk tube or the like may be used; and the reaction is preferably performed in an argon atmosphere.

Examples of the compound containing the transition metal element include alkoxides of transition metal elements, such as zirconium propoxide, zirconium n-butoxide, zirconium 2-ethylhexoxide, hafnium propoxide, hafnium n-butoxide, hafnium 2-ethylhexoxide, titanium propoxide, titanium n-butoxide, and titanium 2-ethylhexoxide.

Examples of the solvent for the alcohol solution include primary alcohols such as methyl alcohol, ethyl alcohol, n-propyl alcohol including 1-propanol or 2-propanol, isopropyl alcohol, n-butyl alcohol, sec-butyl alcohol, tert-butyl alcohol, isobutyl alcohol, hexyl alcohol such as n-hexyl alcohol, heptyl alcohol such as n-heptyl alcohol, octyl alcohol such as n-octyl alcohol, and decanol such as n-decanol; glycol-based solvents such as ethylene glycol, diethylene glycol, triethylene glycol, 1,2-propylene glycol, 1,3-propylene glycol, 1,2-butylene glycol, 1,3-butylene glycol, and 1,4-butylene glycol; alkylene glycol monoalkyl ether-based solvents such as ethylene glycol monomethyl ether, propylene glycol monomethyl ether (PGME; also known as 1-methoxy-2-propanol), ethylene glycol monoethyl ether, propylene glycol monoethyl ether, diethylene glycol monomethyl ether, and triethylene glycol monoethyl ether; glycol ether-based solvents such as methoxymethyl butanol and propylene glycol dimethyl ether; and phenol-based solvents such as phenol and cresol. Among these, primary alcohols are preferred, primary alcohols having 1 to 10 carbon atoms are more preferred, and primary alcohols having 1 to 5 carbon atoms are even more preferred.

Examples of the carboxylic acid having a ligand structure represented by the general formula (1) include carboxylic acids having the following structures. Specific examples thereof include 5-norbornene-2-carboxylic acid.

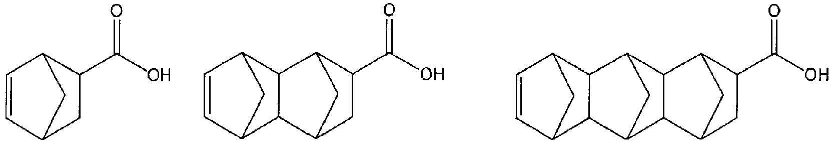

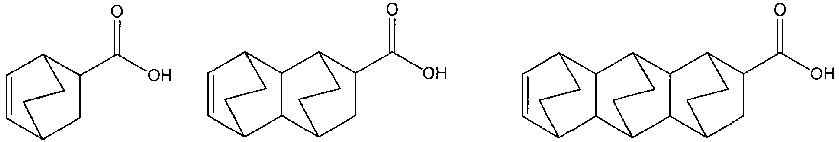

Examples of the carboxylic acid having a ligand structure represented by the general formula (2) include carboxylic acids having the following structures. Specific examples thereof include 3-cyclohexene-1-carboxylic acid.

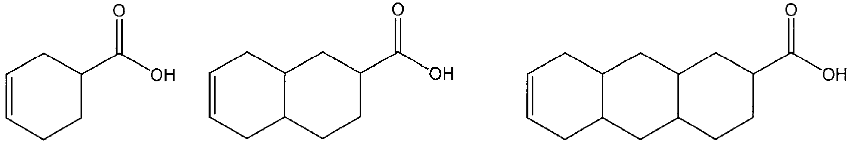

The weight ratio of the alcohol solution of the alkoxide containing a transition metal element to the carboxylic acid having a structure represented by the general formula (1) or (2) is preferably 1:2 to 1:20, more preferably 1:3 to 1:10.

The present poor metal complex compound can be produced, for example, by reacting a carboxylic acid having a structure represented by the general formula (1) or (2) with a poor metal compound using an aprotic solvent. Preferably, it can be produced by reacting a carboxylic acid having a structure represented by the general formula (1) or (2) with a phenyl compound of a poor metal element using an aprotic solvent.

More specifically, for example, a carboxylic acid having a structure represented by the general formula (1) or (2), a phenyl compound of a poor metal element, and an aprotic solvent are mixed in a reaction vessel, and the mixture is heated and stirred for 1 to 24 hours, preferably 4 to 12 hours.

The mixture is then cooled to room temperature, the solvent is removed under vacuum, a poor solvent such as methanol is added, and the precipitated crystals are filtered to obtain the present poor metal complex compound.

Specifically, the temperature for heating and stirring is preferably 50°C to 100°C, more preferably 70°C to 100°C.

The reaction vessel is preferably a sealed vessel, and for small amounts, for example, a Schlenk tube or the like may be used; and the reaction is preferably performed in an argon atmosphere.

Examples of the poor metal include bismuth, and examples of the phenyl compound of the poor metal element include triphenylbismuth.

Examples of the aprotic solvent include aromatic hydrocarbon-based solvents such as toluene and xylene, aliphatic hydrocarbon-based solvents such as pentane, hexane, cyclohexane, octane, decane, and dodecane, ketone-based solvents such as acetone, 2-heptanone, and cyclohexanone, and ether-based solvents such as diethyl ether, isopropyl ether, and tetrahydrofuran.

Examples of the carboxylic acid having a ligand structure represented by the general formula (1) or (2) include carboxylic acids having structures exemplified in the method for producing the present cluster compound described above.

The weight ratio of the poor metal element to the carboxylic acid having a structure represented by the general formula (1) or (2) is preferably 1:2 to 1:20, more preferably 1:3 to 1:10.

### [Photosensitive Composition]

A photosensitive composition according to one embodiment of the present invention (hereinafter also referred to as "the photosensitive composition according to the first embodiment") contains the present cluster compound. The photosensitive composition according to the first embodiment may contain only one type of the present cluster compound, or may contain two or more types thereof.

The content of the present cluster compound in the photosensitive composition according to the first embodiment is generally 50% by mass to 100% by mass, preferably 70% by mass to 100% by mass, and particularly preferably 80% by mass to 100% by mass, relative to the total of components of the photosensitive composition excluding a solvent.

When the content of the present cluster compound in the photosensitive composition according to the first embodiment is equal to or more than the lower limit, good exposure sensitivity can be achieved.

A photosensitive composition according to another embodiment of the present invention (hereinafter also referred to as "the photosensitive composition according to the second embodiment") contains a compound composed of a poor metal element and a carboxy ligand including an alicyclic structure having a double bond. The photosensitive composition according to the second embodiment may contain only one type of a compound composed of a poor metal element and a carboxy ligand including an alicyclic structure having a double bond, or may contain two or more types thereof.

The content of the compound composed of a poor metal element and a carboxy ligand including an alicyclic structure having a double bond in the photosensitive composition according to the second embodiment is generally 50% by mass to 100% by mass, preferably 70% by mass to 100% by mass, and particularly preferably 80% by mass to 100% by mass, relative to the total of components of the photosensitive composition excluding a solvent.

When the content of the compound composed of a poor metal element and a carboxy ligand including an alicyclic structure having a double bond in the photosensitive composition according to the second embodiment is equal to or more than the lower limit, good exposure sensitivity can be achieved.

### [Photoacid Generator]

Each of the photosensitive compositions according to the first and second embodiments (hereinafter also referred to as "the present photosensitive composition") can also function by containing, together with the present cluster compound or the present poor metal complex compound, a photoacid generator that generates an acid upon the action of chemical radiation.

Examples of the chemical radiation include emission ray spectra of mercury lamps, far ultraviolet rays represented by excimer lasers, extreme ultraviolet (EUV) rays, X-rays, and electron beams; and from the viewpoint of resolution, it is preferred that the exposure wavelength is short, and extreme ultraviolet (EUV) rays emitting light having a wavelength of 6.5 to 13.5 nm are preferred.

The photoacid generator that generates an acid by such chemical radiation is not particularly limited as long as it is a known photoacid generator, and is preferably a compound that generates an organic acid, such as at least one of sulfonic acid, bis(alkylsulfonyl)imide, and tris(alkylsulfonyl)methide, upon irradiation with chemical radiation.

The photoacid generator can be used alone or in combination with two or more types. In the case where two or more types of photoacid generators are used in combination, embodiments such as (1) the use of two types of photoacid generators having different acid strengths in combination, or (2) the use of two types of photoacid generators having different sizes (molecular weight or number of carbon atoms) of generated acid in combination, are preferred.

Examples of the embodiment (1) include the use of a sulfonic acid generator having fluorine and a tris(fluoroalkylsulfonyl)methide acid generator in combination, the use of a sulfonic acid generator having fluorine and a sulfonic acid generator having no fluorine in combination, and the use of an alkyl sulfonic acid generator and an aryl sulfonic acid generator in combination.

Examples of the embodiment (2) include the use of two different acid generators each having 4 or more carbon atoms in the generated acid anion in combination.

In particular, the present photosensitive composition is preferably a photosensitive composition used for chemical radiation, because the development speed in a developer changes if it is a photosensitive composition that reacts with chemical radiation, and a pattern can be formed after a certain time of development.

For chemical radiation, shorter wavelength light is preferred because of the higher resolution that can be achieved, and light having a wavelength of 6.5 to 13.5 nm, i.e., extreme ultraviolet (EUV) rays, is preferred.

In other words, the present photosensitive composition is preferably a photosensitive composition that reacts with light having a wavelength of 6.5 to 13.5 nm.

The present photosensitive compound reacts with light even when used alone in the photosensitive composition. The photoacid generator, when it is added, acts synergistically with the present cluster compound in the photosensitive composition for being reacted with light, so that the photosensitivity of the present cluster compound can be increased. Therefore, if a photosensitive composition composed only of the present cluster compound does not have sufficient photosensitivity for the required specifications, it is preferable to add a photoacid generator.

In addition, the photoacid generator, when it is added, acts synergistically with the "compound composed of a poor metal element and a carboxy ligand including an alicyclic structure having a double bond" in the photosensitive composition according to the second embodiment for being reacted with light, to thereby increase the photosensitivity of the compound. Therefore, if a photosensitive composition composed only of the compound does not have sufficient photosensitivity for the required specifications, it is preferable to add a photoacid generator.

When the present photosensitive composition contains a photoacid generator, the content of the photoacid generator in the present photosensitive composition (the total amount if a plurality of photoacid generators are used in combination) is preferably 0.1% by mass to 30% by mass, more preferably 0.5% by mass to 20% by mass, and even more preferably 1% by mass to 15% by mass, based on the total of the components of the photosensitive composition excluding a solvent.

When the content of the photoacid generator in the present photosensitive composition is equal to or more than the lower limit, the effect of increasing the photosensitivity can be achieved, while when the content is equal to or less than the upper limit, good film-forming properties based on the photosensitive compound according to the present invention can be achieved because it is less affected by the poor film-forming properties of the photoacid generator, which are preferred.

### [Solvent]

The present photosensitive composition generally contains a solvent for preparation.

The solvent for preparing the photosensitive composition is not particularly limited as long as it dissolves each component, and examples thereof include alkylene glycol monoalkyl ether carboxylates (such as propylene glycol monomethyl ether acetate (PGMEA; 1-methoxy-2-acetoxypropane)), alkylene glycol monoalkyl ethers (such as propylene glycol monomethyl ether (PGME; 1-methoxy-2-propanol)), alkyl lactate esters (such as ethyl lactate and methyl lactate), cyclic lactones (such as γ-butyrolactone, preferably having 4 to 10 carbon atoms), linear or cyclic ketones (such as 2-heptanone and cyclohexanone, preferably having 4 to 10 carbon atoms), alkylene carbonates (such as ethylene carbonate and propylene carbonate), alkyl carboxylic acid (preferably alkyl acetates such as butyl acetate), alkyl alkoxy acetates (ethyl ethoxypropionate), alkyl amides (N,N-dimethylformamide), and alkyl sulfoxides (dimethyl sulfoxide). Examples of other usable solvents include those described in paragraphs [0244] and following of U.S. Patent Application Publication No. 2008/0248425 A1.

Among these, N,N-dimethylformamide, dimethyl sulfoxide, alkylene glycol monoalkyl ether carboxylates, and alkylene glycol monoalkyl ethers are preferred.

These solvents may be used alone or in combination of two or more types. In the case where two or more types are mixed, it is preferable to mix a solvent having a hydroxyl group with a solvent having no hydroxyl group.

As the solvent having a hydroxyl group, alkylene glycol monoalkyl ethers are preferred; and as the solvent having no hydroxyl group, alkylene glycol monoalkyl ether carboxylate, N,N-dimethylformamide, and dimethyl sulfoxide are preferred.

The content of the solvent in the total amount of the present photosensitive composition can be adjusted as appropriate according to the film thickness of a pattern to be formed or other factors; and in general, the content is adjusted such that the total concentration of the components of the photosensitive composition excluding the solvent is 0.5% by mass to 30% by mass, preferably 1.0% by mass to 20% by mass, more preferably 1.5% by mass to 10% by mass, and even more preferably 1.5% by mass to 5% by mass.

### [Surfactant]

Preferably, the present photosensitive composition further contains a surfactant. As the surfactant, fluorine-based and/or silicon-based surfactants are preferred.

Examples of the surfactants that fall into these categories include MEGAFACE F176 and MEGAFACE R08 manufactured by DIC Corp., PF656 and PF6320 manufactured by OMNOVA, Troysol S-366 manufactured by Troy Chemical Industries, FLUORAD FC430 manufactured by Sumitomo 3M Limited, and Polysiloxane Polymer KP-341 manufactured by Shin-Etsu Chemical Co., Ltd.

In addition, surfactants other than the fluorine-based and/or silicone-based surfactants can be used. Specific examples thereof include polyoxyethylene alkyl ethers and polyoxyethylene alkyl aryl ethers.

Other known surfactants can also be used as appropriate. Examples of other usable surfactants include those described in paragraphs [0273] and following of U.S. Patent Application Publication No. 2008/0248425 A1.

These surfactants may be used alone or in combination of two or more types.

The content of the surfactant is preferably 0.0001% by mass to 2% by mass, more preferably 0.001% by mass to 1% by mass, relative to the total of the components of the photosensitive composition excluding a solvent.

### [Resin]

The present photosensitive composition, although capable of forming a pattern by itself, may also contain a resin material in addition to the present cluster compound and the compound composed of a poor metal element and a carboxy ligand including an alicyclic structure having a double bond. The resin material is not particularly limited as long as it dissolves in a solvent, and examples thereof include novolac resins, styrene resins, and acrylic resins, which may be used alone or in combination of two or more types; and the resin material may contain groups such as a dissolution inhibiting group that decomposes and a crosslinking group that crosslinks, due to chemical active species such as acids and radicals, in the molecular structure, and may be a copolymer resin of two or more types. Examples of the dissolution inhibiting group that decomposes due to chemical active species such as acids and radicals include alkoxycarbonyl groups and acetal groups. Examples of the crosslinking group that crosslinks due to chemical active species such as acids and radicals include vinyl groups, carbodiimide groups, N-hydroxyester groups, imide ester groups, maleimide groups, haloacetyl groups, pyridyl disulfide groups, hydrazide groups, alkoxyamino groups, and diazirine groups.

### [Other Additives]

The present photosensitive composition may appropriately contain, in addition to the components described above, a carboxylic acid, a carboxylic acid onium salt, a dissolution inhibiting compound having a molecular weight of 3,000 or less as described in Proceeding of SPIE, 2724,355 (1996) or the like, a dye, a plasticizer, a photosensitizer, a light absorber, a crosslinking agent, an antioxidant, and the like.

In particular, a carboxylic acid is preferably used for improving performance. Preferred examples of the carboxylic acid include aromatic carboxylic acids such as a benzoic acid and a naphthoic acid.

The content of the carboxylic acid is preferably 0.01% by mass to 10% by mass, more preferably 0.01% by mass to 5% by mass, and even more preferably 0.01% by mass to 3% by mass, relative to the total of the components of the photosensitive composition excluding a solvent.

### [Method for Producing Present Photosensitive Composition]

The present photosensitive composition can be produced by dissolving the present cluster compound or the present poor metal complex compound, and a photoacid generator and other components, which are used as necessary, in a solvent for preparing a solution, and filtering the solution through a filter as necessary. The filter is preferably a filter made of polytetrafluoroethylene, polyethylene, or nylon having a pore size of 0.2 µm or less, preferably 0.1 µm or less, and more preferably 0.05 µm or less.

### [Pattern Forming Method]

A pattern forming method according to one embodiment of the present invention (hereinafter also referred to as "the present pattern forming method") includes a step of forming a photosensitive layer on a substrate using the present photosensitive composition, a step of irradiating a predetermined area of the photosensitive layer with chemical radiation to perform pattern exposure, and a step of developing the photosensitive layer after exposure with a developer to selectively remove the exposed or unexposed area of the photosensitive layer.

### [Step of Forming Photosensitive Layer]

The photosensitive layer can be formed by coating the present photosensitive composition on a substrate (e.g., silicon, silicon dioxide coating) such as that used in the production of integrated circuit elements by a suitable coating method such as a spinner, and then drying at 50°C to 150°C.

In this step, a commercially available inorganic or organic anti-reflective film can be used as necessary. In addition, an anti-reflective film can be coated on a lower layer of the resist.

### [Exposure Step]

In the present invention, the term "exposure" includes not only exposure by far ultraviolet rays represented by mercury lamps and excimer lasers, X-rays, extreme ultraviolet (EUV) rays, and the like, but also lithography by particle beams such as electron beams and ion beams, unless otherwise specified.

The exposure can be performed by irradiating a predetermined area of the formed photosensitive layer with chemical radiation through a predetermined mask to perform pattern exposure, or by irradiating the area with electron beams to lithograph without using a mask (direct lithography) and to perform pattern exposure.

The chemical radiation is not particularly limited, and examples thereof include KrF excimer lasers, ArF excimer lasers, extreme ultraviolet (EUV) rays, and electron beams, of which extreme ultraviolet (EUV) rays and electron beams are preferred, and as described above, extreme ultraviolet (EUV) rays emitting light having a wavelength of 6.5 to 13.5 nm are preferred.

After the exposure, baking (heating) may or may not be performed prior to development.

When baking (heating) is performed, the heating temperature is preferably 50°C to 150°C, more preferably 60°C to 150°C, and even more preferably 80°C to 140°C.

When baking (heating) is performed, the heating time is preferably 30 to 300 seconds, more preferably 30 to 180 seconds, and even more preferably 30 to 90 seconds.

Heating may be performed by a means provided in a normal exposure/developing machine, or by using a hot plate or the like.

### [Development Step]

After exposure, the photosensitive layer is developed using a developer to selectively remove the exposed or unexposed area.

### <Developer>

As the developer, it is preferable to use an organic solvent, preferably an organic solvent having a vapor pressure of 5 kPa or less at 20°C, more preferably 3 kPa or less, and particularly preferably 2 kPa or less. By adjusting the vapor pressure of the organic solvent to 5 kPa or less, evaporation of the developer on the substrate or in the developing cup is suppressed, and the temperature uniformity within the surface of the pattern-formed substrate is improved, resulting in good dimensional uniformity within the surface of the pattern-formed substrate.

### As the organic solvent used as the developer, various organic solvents can be used, and for example, at least one solvent selected from ester-based solvents, ketone-based solvents, alcohol-based solvents, amide-based solvents, sulfoxide-based solvents, ether-based solvents, and hydrocarbon-based solvents can be used.

In particular, it is preferred that the developer contains at least one solvent selected from amide-based solvents, ketone-based solvents, ester-based solvents, alcohol-based solvents, and ether-based solvents.

Examples of the ester-based solvents include alkyl carboxylic acid-based solvents such as methyl acetate, butyl acetate, ethyl acetate, isopropyl acetate, amyl acetate, ethyl-3-ethoxypropionate, propylene glycol diacetate, 3-methoxybutyl acetate, 3-methyl-3-methoxybutyl acetate, methyl formate, ethyl formate, butyl formate, propyl formate, ethyl lactate, butyl lactate, and propyl lactate; and alkylene glycol monoalkyl ether carboxylate-based solvents such as propylene glycol monomethyl ether acetate (PGMEA; also known as 1-methoxy-2-acetoxypropane), ethylene glycol monoethyl ether acetate, diethylene glycol monobutyl ether acetate, diethylene glycol monoethyl ether acetate, and propylene glycol monoethyl ether acetate. Among these, butyl acetate, amyl acetate, ethyl lactate, and propylene glycol monomethyl ether acetate are more preferred.

Examples of the ketone-based solvents include 1-octanone, 2-octanone, 1-nonanone, 2-nonanone, acetone, 4-heptanone, 1-hexanone, 2-hexanone, diisobutyl ketone, cyclopentanone, cyclohexanone, methylcyclohexanone, phenylacetone, methyl ethyl ketone, methyl amyl ketone, methyl isobutyl ketone, acetylacetone, acetonylacetone, ionone, diacetonyl alcohol, acetyl carbinol, acetophenone, methyl naphthyl ketone, isophorone, and propylene carbonate. Among these, alkyl ketone-based solvents, such as methyl isobutyl ketone, methyl amyl ketone, cyclopentanone, and cyclohexanone, are more preferred.

Examples of the alcohol-based solvents include alcohols such as methyl alcohol, ethyl alcohol, n-propyl alcohol including 1-propanol or 2-propanol, isopropyl alcohol, n-butyl alcohol, sec-butyl alcohol, tert-butyl alcohol, isobutyl alcohol, hexyl alcohol such as n-hexyl alcohol, heptyl alcohol such as n-heptyl alcohol, octyl alcohol such as n-octyl alcohol, and decanol such as n-decanol; glycol-based solvents such as ethylene glycol, diethylene glycol, triethylene glycol, 1,2-propylene glycol, 1,3-propylene glycol, 1,2-butylene glycol, 1,3-butylene glycol, and 1,4-butylene glycol; alkylene glycol monoalkyl ether-based solvents such as ethylene glycol monomethyl ether, propylene glycol monomethyl ether (PGME; also known as 1-methoxy-2-propanol), ethylene glycol monoethyl ether, propylene glycol monoethyl ether, diethylene glycol monomethyl ether, and triethylene glycol monoethyl ether; glycol ether-based solvents such as methoxymethyl butanol and propylene glycol dimethyl ether; and phenol-based solvents such as phenol and cresol. Among these, 1-hexanol, 2-hexanol, 1-octanol, 2-ethyl-hexanol, propylene glycol monomethyl ether, and cresol are more preferred.

Examples of the amide-based solvents include N-methyl-2-pyrrolidone, N,N-dimethylacetamide, N,N-dimethylformamide, hexamethylphosphoric triamide, and 1,3-dimethyl-2-imidazolidinone.

Examples of the sulfoxide-based solvents include dimethyl sulfoxide.

Examples of the ether-based solvents include dioxane, tetrahydrofuran, and tetrahydropyran, in addition to the alkylene glycol monoalkyl ether-based solvents and the glycol ether-based solvents described above.

Examples of the hydrocarbon-based solvents include aromatic hydrocarbon solvents such as toluene and xylene; and aliphatic hydrocarbon solvents such as pentane, hexane, octane, decane, and dodecane.

It is preferred that the developer contains one or more solvents selected from alkylene glycol monoalkyl ether carboxylate-based solvents, alkylene glycol monoalkyl ether-based solvents, alkyl carboxylic acid-based solvents, and alkyl ketone-based solvent; and it is more preferred that the developer contains one or more solvents selected from dimethylformamide, dimethyl sulfoxide, tetrahydrofuran, ethylene glycol, methyl alcohol, ethyl alcohol, 1-propanol, and 2-propanol.

As the developer, it is preferable to use a developer containing at least one organic solvent selected from the group consisting of ester-based solvents having no hydroxyl group in the molecule, ketone-based solvents having no hydroxyl group in the molecule, ether-based solvents having no hydroxyl group in the molecule, amide-based solvents, and sulfoxide-based solvents.

The organic solvent used as the developer in the present invention is preferably an organic solvent having a solubility parameter (SP value) of 7.5 to 11. An organic solvent having a solubility parameter of 7.5 or more is preferred because the development speed of the dissolved portion increases, while an organic solvent having a solubility parameter of 11 or less is preferred because the development speed of the pattern-formed portion can be suppressed. The solubility parameter of the organic solvent in the developer is more preferably 8 to 11.

In the present invention, the solubility parameter (SP value) is calculated by the method proposed by Fedors et al. Specifically, it is the value determined by reference to "POLYMER ENGINEERING AND SCIENCE, FEBRUARY, 1974, Vol. 14, No.2, ROBERTF. FEDORS. (P.147 - 154)". The SP value is a physical property value determined by the content of hydrophobic and hydrophilic groups in the molecule, and when a mixed solvent is used, it refers to the value of the mixture.

Examples of the organic solvent that satisfies the above SP value include diethylene glycol monomethyl ether (SP value = 10.7), triethylene glycol monomethyl ether (SP value = 10.7), ethylene glycol monoisopropyl ether (SP value = 10.9), ethylene glycol monobutyl ether (SP value = 10.2), diethylene glycol monobutyl ether (SP value = 10.0), triethylene glycol monobutyl ether (SP value = 10.0), ethylene glycol monoisobutyl ether (SP value = 9.1), ethylene glycol monohexyl ether (SP value = 9.9), diethylene glycol monohexyl ether (SP value = 9.7), diethylene glycol mono 2-ethylhexyl ether (SP value = 9.3), ethylene glycol monoallyl ether (SP value = 10.8), ethylene glycol monophenyl ether (SP value = 10.8), ethylene glycol monobenzyl ether (SP value = 10.9), propylene glycol monomethyl ether (SP value = 10.0), dipropylene glycol monomethyl ether (SP value = 9.7), tripropylene glycol monomethyl ether (SP value = 9.4), propylene glycol monopropyl ether (SP value = 9.6), dipropylene glycol monopropyl ether (SP value = 9.8), propylene glycol monobutyl ether (SP value = 9.0), dipropylene glycol monobutyl ether (SP value = 9.6), ethylene glycol monomethyl ether acetate (SP value = 10.0), ethylene glycol monoethyl ether acetate (SP value = 9.6), ethylene glycol monobutyl ether acetate (SP value = 8.9), diethylene glycol monoethyl ether acetate (SP value = 9.4), diethylene glycol monobutyl ether acetate (SP value = 9.0), propylene glycol monomethyl ether acetate (SP value = 9.4), propylene glycol monoethyl ether acetate (SP value = 9.0), and dipropylene glycol monomethyl ether acetate (SP value = 9.2).

The organic solvents described above may be used in combination, or may be used in combination with a solvent other than the above or with water.

The concentration of the organic solvent (the total if a plurality of organic solvents are mixed) in the developer is preferably 50% by mass or more, more preferably 70% by mass or more, and even more preferably 90% by mass or more. It is particularly preferred that the developer is substantially composed only of organic solvents. The term "substantially composed only of organic solvents" includes cases where trace amounts of surfactants, antioxidants, stabilizers, defoaming agents, and the like are contained.

The water content in the developer is preferably 10% by mass or less, more preferably 5% by mass or less, and particularly preferably 3% by mass or less; and it is most preferred that the developer contains substantially no water. By adjusting the water content to 10% by mass or less, good development characteristics can be achieved.

An appropriate amount of a surfactant may be added to the developer used in the present invention, as necessary.

As the surfactant, the same surfactants as those described above used in the photosensitive composition according to the present invention may be used.

The amount of the surfactant used is generally 0.001% by mass to 5% by mass, preferably 0.005% by mass to 2% by mass, and more preferably 0.01% by mass to 0.5% by mass, relative to the total amount of the developer.

### <Development Method>

Examples of the development method that can be applied include a method of immersing a substrate into a tank filled with a developer for a certain period of time (dip method), a method of piling up a developer on the surface of a substrate by surface tension and leaving it there for a certain period of time (paddle method), a method of spraying a developer on the surface of a substrate (spray method), and a method of continuously dispensing a developer while scanning a developer dispensing nozzle at a constant speed onto a substrate rotating at a constant speed (dynamic dispense method).

After the development step, a step of stopping the development while replacing the solvent with other solvents may be performed.

The development time is preferably a time required for the present cluster compound and the like in the unexposed or exposed area of the photosensitive layer to be sufficiently dissolved, and is generally preferably 10 to 300 seconds, more preferably 20 to 120 seconds.

The temperature of the developer is preferably 0°C to 50°C, more preferably 15°C to 35°C.

The amount of the developer can be adjusted as appropriate according to the development method.

### [Rinsing Step]

The present pattern forming method may include a step of washing using a rinse solution containing an organic solvent after the development step.

### <Rinse Solution>

The organic solvent used in the rinse solution preferably has a vapor pressure of 0.05 kPa or more and 5 kPa or less at 20°C, more preferably 0.1 kPa or more and 5 kPa or less, and most preferably 0.12 kPa or more and 3 kPa or less. By adjusting the vapor pressure of the organic solvent used in the rinse solution to 0.05 kPa or more and 5 kPa or less, the temperature uniformity within the wafer surface is improved, and swelling caused by the penetration of the rinse solution is suppressed, thereby improving the dimensional uniformity within the wafer surface.

As the rinse solution, various organic solvents can be used, and for the present cluster compound, a rinse solution containing at least one organic solvent selected from hydrocarbon-based solvents, ketone-based solvents, ester-based solvents, alcohol-based solvents, amide-based solvents, and ether-based solvents, or water is preferably used.

More preferably, after the development, a step of washing is performed using a rinse solution containing at least one organic solvent selected from ketone-based solvents, ester-based solvents, alcohol-based solvents, amide-based solvents, and hydrocarbon-based solvents. Even more preferably, after the development, a step of washing is performed using a rinse solution containing at least one organic solvent selected from the group consisting of alcohol-based solvents and hydrocarbon-based solvents.

Specific examples of the ketone-based solvents, ester-based solvents, alcohol-based solvents, amide-based solvents, ether-based solvents, and hydrocarbon-based solvents used as the rinse solution are the same as those described above for the developer.

Most preferably, a rinse solution containing at least one organic solvent selected from the group consisting of monohydric alcohol-based solvents, hydrocarbon-based solvents, and amide-based solvents is used.

Examples of the monohydric alcohol-based solvents used in the rinsing step after development include linear, branched, and cyclic monohydric alcohols, and specific examples thereof include 1-butanol, 2-butanol, 3-methyl-1-butanol, tert-butyl alcohol, isopropyl alcohol, cyclopentanol, and cyclohexanol, of which 1-butanol, 2-butanol, 3-methyl-1-butanol, and isopropyl alcohol are preferred.

Examples of the hydrocarbon-based solvents include aromatic hydrocarbon-based solvents such as toluene and xylene, and aliphatic hydrocarbon-based solvents such as octane, decane, and dodecane.

Examples of the amide-based solvents include N,N-dimethylformamide.

The components described above may be used in combination in plural numbers, or may be used in combination with organic solvents other than those described above.

The organic solvent may be mixed with water. However, the water content in the rinse solution is generally 30% by mass or less, preferably 10% by mass or less, more preferably 5% by mass or less, and particularly preferably 3% by mass or less. It is most preferred that the rinse solution contains no water. By adjusting the water content to 30% by mass or less, good development characteristics can be achieved.

An appropriate amount of a surfactant may be added to the rinse solution.

As the surfactant, the same surfactants as those used in the photosensitive composition described above can be used; and the amount of the surfactant used is generally 0.001% by mass to 5% by mass, preferably 0.005% by mass to 2% by mass, and more preferably 0.01% by mass to 0.5% by mass, relative to the total amount of the rinse solution.

### <Rinsing Method>

In the rinsing step, the developed pattern-formed substrate is washed using the rinse solution containing organic solvents described above.

The washing method is not particularly limited, and examples of the method that can be applied include a method of continuously coating a rinse solution onto a substrate rotating at a constant speed (spin coating method), a method of immersing a substrate into a tank filled with a rinse solution for a certain period of time (dip method), and a method of spraying a rinse solution onto the surface of a substrate (spray method). Among these, it is preferable to perform washing by the spin coating method, and then to spin the substrate at a number of rotations of 2,000 to 4,000 rpm after the washing to remove the rinse solution from the substrate. The substrate rotation time can be set within a range where the rinse solution is removed from the substrate according to the number of rotations, and is generally 10 seconds to 3 minutes. It is preferable to perform rinsing at room temperature.

The rinsing time is preferably such that no developing solvent remains on the substrate, and is generally preferably 10 to 300 seconds. It is more preferably 20 to 120 seconds.

The temperature of the rinse solution is preferably 0°C to 50°C, more preferably 15°C to 35°C.

The amount of the rinse solution can be adjusted as appropriate according to the rinsing method.

### [Post-Treatment Step]

After developing or rinsing, a treatment may be performed in which the developer or rinse solution adhering on the pattern is removed by using a supercritical fluid.

In addition, after development, rinsing, or supercritical fluid treatment, a heat treatment may be performed to remove the solvent remaining on the pattern. The heating temperature and time are not particularly limited as long as a good resist pattern is obtained, and are generally 40°C to 160°C for 10 seconds to 3 minutes. The heat treatment may be performed several times.

### [Applications]

The present photosensitive composition and the present pattern forming method are suitable for use in the production of semiconductor fine circuits, such as the production of ultra-LSIs and high-capacity microchips. In the production of semiconductor fine circuits, a pattern-formed resist film is subjected to circuit formation and etching, and the remaining resist film portion is finally removed using a solvent or the like, so that, unlike so-called permanent resists used in printed circuit boards or the like, no resist film derived from the present photosensitive composition remains on the final product such as a microchip.

### EXAMPLES

Examples of the present invention are described below. However, the present invention is not limited to the following Examples. The terms "parts" and "%" in Examples are on a mass basis unless otherwise specified.

The following compounds 1 to 3 were prepared and obtained as Examples 1 to 3, respectively. The composition and ligand structure of each compound were measured by ¹H-NMR.

### <Compound 1>

In a reaction vessel, 5.3 mL (17.0 mmol) of a zirconium propoxide solution (70% propanol solution), 4.9 g (35.6 mmol) of 5-norbornene-2-carboxylic acid, and 10 mL of 1-propanol were mixed, the mixture was stirred at room temperature for 1 hour, and then the solvent was removed under reduced pressure to prepare 7.0 g of a pale yellow amorphous solid. The resulting substance was analyzed by NMR and found to have a structure represented by the following chemical formula 1.
¹H-NMR(CDCl₃, ppm): 6.5-5.8 (br,2H), 3.3-2.0 (br,3H), 2.0-1.1 (br,4H)

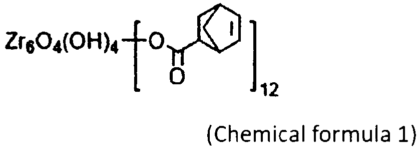

### <Compound 2>

In a reaction vessel, 5.9 mL (18.9 mmol) of a zirconium propoxide solution (70% propanol solution), 5.0 g (39.6 mmol) of 3-cyclohexene-1-carboxylic acid, and 10 mL of 1-propanol were mixed, the mixture was stirred at room temperature for 1 hour, and then the solvent was removed under reduced pressure to prepare 6.5 g of a pale yellow oily substance. The resulting substance was analyzed by NMR and found to have a structure represented by the following chemical formula 2.
¹H-NMR (CDCl₃, ppm): 5.8-5.4 (br,2H), 2.6-1.8 (br,5H), 1.8-1.4 (br,2H)
ESI-MS: 2035.05 (isotope pattern: C₇ₒH₉₆O₂₈Zr₆)

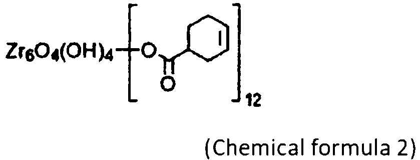

### <Compound 3>

In a reaction vessel, 1.18 g (2.68 mmol) of triphenylbismuth, 1.00 g (8.05 mmol) of 3-cyclohexenecarboxylic acid, and 3 ml of dehydrated toluene were mixed under a nitrogen atmosphere, and the mixture was heated and stirred at 75°C for 8 hours. The mixture was then cooled to room temperature, the solvent was concentrated under reduced pressure, 10 ml of methanol was added to the residue, and the precipitate was filtered to prepare a white powder (0.52 g). The resulting solid was analyzed by NMR and found to be the following compound 3.
¹H-NMR(CDCl₃, ppm): 5.7 (br,2H), 2.5-1.6 (m,7H)
ESI-MS: 607.20

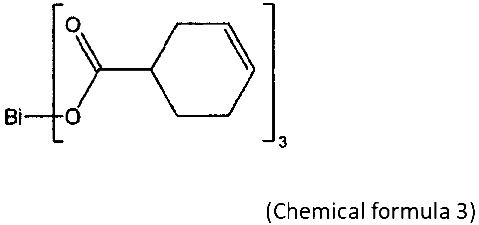

### <Photosensitive Composition (Resist Solution) Preparation>

Each of the compounds 1 to 3 was dissolved in a solvent listed in Table 1 at a concentration of 3% by mass, and filtered through a 0.2 µm filter to prepare a resist solution.

### <Resist Film Formation and Patterning>

The prepared resist solution was spin-coated onto a pattern- formed substrate (silicon wafer) to form a resist film having a thickness of approximately 40 nm. The resulting resist film was baked at 90°C for 90 seconds, and then patterned using an electron beam lithography system (electron beam acceleration voltage: 100 keV).

### <Development>

After patterning, the compounds 1 and 2 were developed (25°C, 60 seconds) with cyclohexanone and the compound 3 was developed (25°C, 30 seconds) with ethyl lactate to obtain negative-type patterns, which were designated as Examples 1 to 3, respectively.

### <Sensitivity Measurement>

Extreme ultraviolet (EUV) exposure was performed at different doses of irradiation, and the film thickness of each developed pattern was measured using a contact step gauge. The dose of EUV irradiation at which the change in film thickness was maximized was determined as an index of sensitivity. The sensitivity is higher when the exposure dose (unit: mJ/cm²) is smaller, and is lower when the exposure dose is larger.

The results are shown in Table 1.

### [Comparative Example 1]

Polystyrene (PS, weight average molecular weight of 4,000) manufactured by Sigma-Aldrich was dissolved in propylene glycol monomethyl ether acetate at a concentration of 2% by mass, and filtered through a 0.2 µm filter to prepare a resist solution.

### [Comparative Example 2]

The following compound 4 having zirconium as the transition metal and a structure derived from methacrylic acid as the ligand was dissolved in N,N-dimethylformamide at a concentration of 3% by mass, and filtered through a 0.2 µm filter to prepare a resist solution.

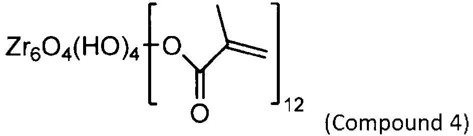

The resist solutions in Comparative Examples 1 and 2 were evaluated in the same manner as in Examples 1 to 3. The results are shown in Table 1.

**[Table 1]**

| | Resist solution composition | | Sensitivity [mJ/cm²] |
|---|---|---|---|
| | Compound type | Solvent | |
| Example 1 | Compound 1 | 2-Heptanone | 30 |
| Example 2 | Compound 2 | 2-Heptanone | 30 |
| Example 3 | Compound 3 | Ethyl lactate | 50 |
| Comparative Example 1 | PS | Propylene glycol monomethyl ether acetate | >100 |
| Comparative Example 2 | Compound 4 | N,N-Dimethylformamide | 15 |

Based on the results in Table 1, it was confirmed that the sensitivity in Examples 1 to 3 was higher than that in Comparative Example 1 because the dose of EUV irradiation at which the change in film thickness was maximized was small.

### <Resolution Evaluation>

The photosensitive compositions in Examples 1 to 3 were evaluated for resolution using an electron beam lithography system. The resolution was evaluated by patterning line-and-space (line : space = 1:1) patterns with half-pitches (hp) of 100 nm and 50 nm, developing the pattern, and observing each pattern using a scanning electron microscope. Those in which a line-and-space pattern of hp 100 nm was observed were rated as "A", those in which a pattern was observed but partially crosslinked were rated as "B", and those in which no pattern was observed were rated as "C".

The results are shown in Table 2.

**[Table 2]**

| | hp 100 nm | hp 50 nm |
|---|---|---|
| Example 1 | A | A |
| Example 2 | A | A |
| Example 3 | A | A |
| Comparative Example 1 | A | C |
| Comparative Example 2 | A | A |

### [Results]

Based on the results of the electron beam (EB) lithography test, in Examples 1 to 3, a line-and-space (L&S, 1:1) pattern of hp (half-pitch) 100 nm was formed, and especially in Examples 1 and 3, a line-and-space (L&S, 1:1) pattern of hp 50 nm was formed despite containing defects. As shown in Table 1, the sensitivity to EUV exposure was also confirmed to be extremely high.

### <Coating Property Evaluation>

Each of the resist solutions in Examples 1 to 3 and Comparative Examples 1 and 2 was spin-coated onto a silicon wafer and baked at 90°C for 90 seconds to form a film. The coating uniformity was visually evaluated according to the criteria of "O: repelling" and "×: no repelling". The results are shown below.

**[Table 3]**

| | Coating uniformity |
|---|---|
| Example 1 | ○ |
| Example 2 | ○ |
| Example 3 | ○ |
| Comparative Example 1 | ○ |
| Comparative Example 2 | × |

As shown in the results above, in Comparative Example 2, it was easy to repel on the silicon wafer and the coating properties were poor. This is presumably because it is poorly soluble in organic solvents and the types of solvents that are soluble are limited. If the coating properties are poor, it is difficult to use the composition in high-yield semiconductor production, which is of little practical use. On the other hand, in Examples 1 to 3, the coating film was uniformly formed without any repelling, thus increasing the yield of semiconductor production, and the practicality was evaluated as high.

Therefore, the compound composed of a carboxy ligand including an alicyclic structure having a double bond and a transition metal or a poor metal element can be practically used as a photoresist suitable for ultra-fine patterns.

## Claims

1. A transition metal cluster compound comprising a transition metal element and a carboxy ligand including an alicyclic structure having a double bond, wherein the compound forms a cluster centered on the transition metal element.

2. The transition metal cluster compound according to claim 1, wherein the carboxy ligand including an alicyclic structure having a double bond comprises a ligand represented by the following general formula (1): 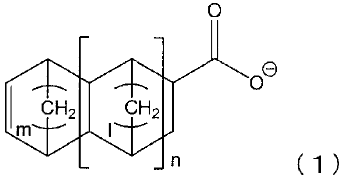 wherein n represents an integer of 0 or 1 or more, and m and 1 each represent an integer of 1 or more and may be the same or different.

3. The transition metal cluster compound according to claim 1, wherein the carboxy ligand including an alicyclic structure having a double bond comprises a ligand represented by the following general formula (2): 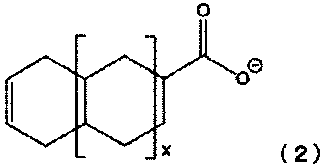 wherein x represents an integer of 0 or 1 or more.

4. The transition metal cluster compound according to claim 1, wherein the transition metal element comprises one or more elements selected from the group consisting of zirconium, hafnium, and titanium.

5. The transition metal cluster compound according to claim 1, comprising 2 or more and 6 or less of the transition metal elements.

6. A method for producing the transition metal cluster compound according to claim 2 or 3, wherein a carboxylic acid having a structure represented by the general formula (1) or (2) is reacted with a compound containing a transition metal element in a solution.

7. A method for producing the transition metal cluster compound according to claim 2 or 3, wherein a carboxylic acid having a structure represented by the general formula (1) or (2) is reacted with a compound containing a transition metal element in a solution in the presence of water.

8. A method for producing the transition metal cluster compound according to claim 2 or 3, wherein a carboxylic acid having a structure represented by the general formula (1) or (2) is mixed into an alcohol solution of a compound containing a transition metal element and reacted in the solution.

9. A method for producing the transition metal cluster compound according to claim 2 or 3, wherein a carboxylic acid having a structure represented by the general formula (1) or (2) is reacted with an alcohol solution of an alkoxide of the transition metal element.

10. A photosensitive composition comprising the transition metal cluster compound according to claim 1.

11. The photosensitive composition according to claim 10, wherein the transition metal cluster compound accounts for 50% by mass or more of components of the photosensitive composition excluding a solvent.

12. The photosensitive composition according to claim 10, wherein the photosensitive composition reacts with light having a wavelength of 6.5 to 13.5 nm.

13. A pattern forming method comprising a step of coating the photosensitive composition according to claim 10 onto a substrate, a step of exposing the composition to chemical radiation, and a step of developing the composition using a developer.

14. The pattern forming method according to claim 10, wherein the developer comprises an organic solvent having a solubility parameter (SP value) of 7.5 to 11.

15. A method for producing a substrate having a patterned layer obtained by the pattern forming method according to claim 10.

16. A complex compound comprising a poor metal element and a carboxy ligand including an alicyclic structure having a double bond.

17. The complex compound according to claim 16, wherein the poor metal element comprises arsenic, antimony, or bismuth.

18. The complex compound according to claim 16, wherein the carboxy ligand including an alicyclic structure having a double bond comprises a ligand represented by the following general formula (1) or (2): 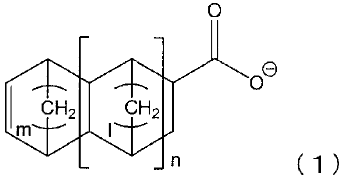
wherein n represents an integer of 0 or 1 or more, and m and 1 each represent an integer of 1 or more and may be the same or different; and 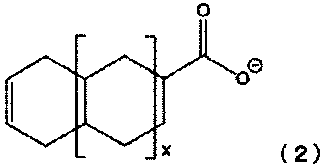
wherein x represents an integer of 0 or 1 or more.

19. The complex compound according to claim 16, comprising a compound represented by the following general formula (3) or (4): 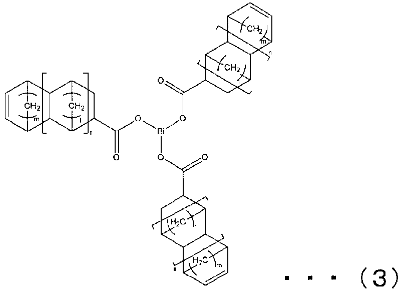
wherein n represents an integer of 0 or 1 or more, and m and 1 each represent an integer of 1 or more and may be the same or different; and 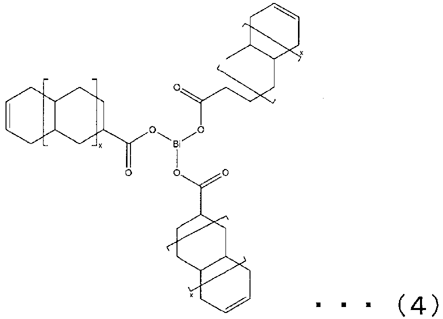
wherein x represents an integer of 0 or 1 or more.

20. A method for producing the complex compound according to claim 18, wherein a carboxylic acid having a structure represented by the general formula (1) or (2) is reacted with a compound containing a poor metal element using an aprotic solvent.

21. A method for producing the complex compound according to claim 18, wherein a carboxylic acid having a structure represented by the general formula (1) or (2) is reacted with a bismuth compound using an aprotic solvent.

22. A photosensitive composition comprising the complex compound according to claim 16.

23. The photosensitive composition according to claim 22, wherein the complex compound accounts for 50% by mass or more of components of the photosensitive composition excluding a solvent.

24. The photosensitive composition according to claim 22, wherein the photosensitive composition reacts with light having a wavelength of 6.5 to 13.5 nm.

25. A pattern forming method comprising a step of coating the photosensitive composition according to claim 22 onto a substrate, a step of exposing the composition to chemical radiation, and a step of developing the composition using a developer.

26. The pattern forming method according to claim 25, wherein the developer comprises an organic solvent having a solubility parameter (SP value) of 7.5 to 11.

27. A method for producing a substrate having a patterned layer obtained by the pattern forming method according to claim 26.

28. A photosensitive composition comprising a compound composed of a transition metal or a poor metal element and of a carboxy ligand including an alicyclic structure having a double bond.

29. A pattern forming method comprising a step of coating the photosensitive composition according to claim 28 onto a substrate, a step of exposing the composition to chemical radiation, and a step of developing the composition using a developer.
